**Europäisches Patentamt**

**European Patent Office**

**Office européen des brevets**

(19)

(11) Veröffentlichungsnummer: **0 121 725**
**B1**

(12) # EUROPÄISCHE PATENTSCHRIFT

(45) Veröffentlichungstag der Patentschrift:
**25.11.87**

(51) Int. Cl.⁴: **A 61 F 5/01,** A 61 F 13/06,
A 61 F 5/14

(21) Anmeldenummer: **84102092.8**

(22) Anmeldetag: **29.02.84**

(54) Vorrichtung zur Anhebung des Vorderfusses.

(30) Priorität: **03.03.83 DE 3307815**

(43) Veröffentlichungstag der Anmeldung:
**17.10.84 Patentblatt 84/42**

(45) Bekanntmachung des Hinweises auf die Patenterteilung:
**25.11.87 Patentblatt 87/48**

(84) Benannte Vertragsstaaten:
**AT CH DE FR GB IT LI NL SE**

(56) Entgegenhaltungen:
**FR-A-523 538**
**FR-A-1 192 109**
**GB-A-739 111**
**US-A-3 527 209**
**US-A-4 280 488**

(73) Patentinhaber: **C. Nicolai GmbH & Co. KG, Kleine Düwelstrasse 21, D-3000 Hannover 1 (DE)**

(72) Erfinder: **Philipp, Alexander, Osterwalder Strasse 3, D-3006 Garbsen 4 (DE)**

(74) Vertreter: **Eikenberg & Brümmerstedt Patentanwälte, Schackstrasse 1, D-3000 Hannover 1 (DE)**

## Beschreibung

Die Erfindung betrifft eine Vorrichtung zur Anhebung des Vorderfußes bei der Funktionsstörung des Peronaeusnervs unter Verwendung einer zwischen dem Vorderfuß und dem Unterschenkel wirkenden elastischen Verbindung mit einstellbarer Vorspannung.

Beim gesunden Menschen ergibt sich ein störungsfreier Bewegungsablauf beim Gehen oder Laufen dadurch, daß wechselweise der Vorderfuß angehoben und abgesenkt wird. Das Anheben des Vorderfußes erfolgt durch die im Fußrücken verlaufenden Muskeln und Bänder, die durch den zum vegetativen Nervensystem gehörenden Personaeusnerv gesteuert werden. Durch eine nach einem Schlaganfall häufig zurückbleibende partielle Nervenlähmung oder durch Folgeschäden eines Unfalls, z. B. einer Unterbrechung des Nervenleitungssystems oder einer Gehirnblutung, kommt es oft zu einer Funktionsstörung des Peronaeusnervs und damit zu neurologischen Ausfällen der fußhebenden Muskeln und Bänder. Bei auf solche Weise behinderten Personen hängt der Vorderfuß aufgrund seines Eigengewichts dann ständig nach unten. Beim Vorsetzen des Fußes während der gehenden oder laufenden Bewegung muß das Knie dann abnorm hochgehoben werden (Steppergang), wenn es nicht zu einem Schleifen der Zehen auf dem Boden und damit zu einem ständigen Stolpern kommen soll.

Eine bekannte Vorrichtung zum Heben des Vorderfußes (Zeitschrift "Medizinische Klinik", 1955, Heft 49, Seite 2094) ist eine unter der Fußsohle und hinter dem Wadenbein angeordnete verhältnismäßig starre Winkelschiene, die den Fuß in seiner Normallage fixiert. Der wadenbeinseitige Schenkel der Winkelschiene wird mit dem Unterschenkel der behinderten Person, beispielsweise durch einen Riemen verbunden. Der fußsohlenseitige Schienenschenkel ist in eine orthopädische Schuheinlage oder direkt in einen orthopädischen Schuh eingearbeitet.

Diese Vorrichtung hat eine Reihe von Nachteilen. Durch die verhältnismäßig starre Winkelschiene ist der Fuß in seiner Lage unveränderbar fixiert. Damit wird das bei einer Funktionsstörung des Peronaeusnervs noch mögliche Absenken des Fußes stark behindert und damit ein ungehindertes Abrollen des Fußes ausgeschlossen.

Ein weiterer Nachteil dieser bekannten Vorrichtung liegt in den unhandlichen Abmessungen der Winkelschiene, wodurch eine Aufnahme der Vorrichtung in einem handelsüblichen Schuh unmöglich ist, so daß von der behinderten Person ausschließlich orthopädische Schuhe verwendet werden müssen. Damit ist eine unbehinderte Fortbewegung der erkrankten Person z. B. im häuslichen Bereich beim Tragen von Sandalen oder Hausschuhen ausgeschlossen.

Schließlich ist diese Vorrichtung auch in kosmetischer Hinsicht sehr nachteilig, da der orthopädische Schuh unmodern und klobig erscheint, so daß sich dieser Schuh gegenüber dem auf dem gesunden Fuß getragenen zweiten Schuh stark abhebt und zudem infolge seines hohen Eigengewichts unbequem zu tragen ist.

Es ist ferner bekannt, (DE-C-349 372) bei einem Stiefel zwischen dem den Vorderfuß umgebenden Teil und dem den Unterschenkel umschließenden Schaft eine elastische Verbindung vorzusehen, die aus einem Riemen und einem Gummiband besteht, wobei die Vorspannung, mit der der Vorderfuß angehoben wird, einstellbar ist.

Auch diese Anordnung hat den Nachteil, daß ein Spezialschuh verwendet werden muß, dessen optische Erscheinung den Krankheitszustand des Trägers sofort erkennen läßt, und der dem Erkrankten eine Hilfe nur dann bietet, wenn er den Schuh auch tatsächlich trägt.

Aus der GB-A- ist eine am Schienbein befestigbare Schiene aus Federmetall bekannt. Die Schiene wird unter der Socke getragen und ist nur mit Schuh wirksam.

Der Erfindung liegt die Aufgabe zugrunde, eine Vorrichtung zu schaffen, die die obengenannten Nachteile nicht aufweist.

Die gestellte Aufgabe wird gemäß der Erfindung dadurch gelöst, daß die Vorrichtung aus einer fest am Fuß anliegenden Socke besteht, auf deren fußrückenseitiger Oberfläche eine Schiene befestigt ist, welche aus einem elastisch nachgiebigem Material dem Fußrücken anatomisch angepaßt gefertigt ist und zwischen Vorderfuß und unterem Schienbeinbereich angeordnet ist, und daß die elastische Verbindung an den Enden der Schiene angreift.

Die erfindungsgemäße Anordnung der Schiene auf der Socke wird der Anatomie des Fußes gerecht, da die mit der Vorrichtung zu ersetzenden fußhebenden und fußsetzenden Bänder ebenfalls auf dem Fußrücken vorhanden sind. Darüber hinaus ist durch die in der Schiene vorhandene Eigenelastizität jederzeit die Möglichkeit gegeben, ein Absenken des Fußes durchzuführen, wodurch die Abrollbewegung des Fußes beim Gehen nicht behindert wird. Da die Vorrichtung mit der Socke eine Einheit bildet, ist eine Benutzung auch ganz ohne Schuhe möglich, wodurch die Bewegung im häuslichen Bereich erleichtert wird. Die Vorrichtung erlaubt aber auch das Tragen von normalem Schuhwerk und ist bequem zu tragen sowie kosmetisch unauffällig.

Vorzugsweise sitzt die Schiene mit ihren beiden Enden in zwei mit der Socke fest vernähten Taschen, wobei sie zweckmäßiger Weise in die Taschen einsteckbar ist. Dadurch wird das Anziehen der Socke erleichtert, denn die Praxis hat gezeigt, daß es einfacher ist, erst die Socke ohne die dabei hinderliche Schiene anzuziehen und erst anschließend die Schiene anzubringen.

Die Socke besteht aus einem elastischen Gewebe, das im Fersenbereich und im Bereich der beiden Taschen verstärkt ist. Dadurch liegt die Socke am Fuß fest an, und die Verstärkungen tragen dazu bei, daß die beim Gehen von der Schiene ausgeübten Kräfte aufgenommen werden können.

In weiterer Ausgestaltung der Erfindung ist die in ansich bekannter Weise als Gummizug ausgebildete elastische Verbindung in einen mittleren Bereich zwischen den Taschen mittels eines Fixierungselements geführt, das beiderseits des Gummizugs mit je einer seitlich abstehenden Lasche versehen ist, wobei der Gummizug oberhalb des Fixierungselements durch ein Klettband verlängert ist, und wobei das freie Ende des Klettbandes um einen Steg, der in die Schiene am schienbeinseitigen Ende eingearbeitet ist, zum Fixierungselement zurückgeführt und daran befestigt ist. Die Umlenkung des Klettbandes an dem Steg und die Verwendung eines Klettbandverschlußes haben den Vorteil, daß der Gummizug durch das um den Steg geführte Klettband bei jeder gewählten Vorspannung fixiert werden kann, da die möglichen Befestigungspunkte über die ganze Länge des Bandes verteilt und nicht auf einen Punkt beschränkt sind.

Zweckmäßigerweise sind die beiden Laschen des Fixierungselements an ihrer der Socke zugewandten Oberfläche mit Klettmaterial versehen und dadurch auf der Socke festlegbar. Hierdurch wird einerseits erreicht, daß das zuvor zwischen den Enden der Schiene sehnenartig gespannte Gummiband am Fußrücken bzw. der Schiene zur Anlage kommt und damit nicht mehr stört, und daß andererseits hierdurch der Gummizug eine zusätzliche Vorspannung erfährt, die den Fuß überstreckt. Diese Überstreckung ist von Vorteil, wenn anschließend ein Schuh angezogen wird, da durch das Eigengewicht des Schuhes diese Überstreckung des Fußes wieder ausgeglichen wird, so daß der Fuß mit dem Schuh wieder seine Normallage einnimmt.

Bei einer anderen Ausführungsform der Erfindung besteht die elastische Verbindung aus zwei Gummibändern, die mit ihrem einen Ende mit seitlichem Abstand voneinander an dem vorderfußseitigen Ende der Schiene starr befestigt sind und an ihrem anderen Ende jeweils eine verstellbare Schnalle mit dem einen Teil eines Druckknopfes aufweisen, der auf die anderen Teile der Druckknöpfe, die an dem schienbeinseitigen Ende der Schiene in seitlichem Abstand voneinander angebracht sind, aufrastbar sind, wobei die beiden Gummibänder vom einen Ende der Schiene zum anderen Ende über Kreuz geführt sind. Diese Ausführungsform hat den Vorteil einer besseren seitlichen Stabilisierung für den Fuß.

Dabei ist vorzugsweise im mittleren Bereich der Schiene eine Öse angebracht, durch die die Gummibänder in unmittelbarer Nähe an der Schiene hindurchgeführt sind. Eine solche Öse hat den Vorteil, daß sie sehr wenig aufträgt.

Die Erfindung wird nachfolgend anhand von in der Zeichnung dargestellten Ausführungsbeispielen näher erläutert. In der Zeichnung zeigen:

Fig. 1 eine Vorderansicht einer Ausführungsform der Vorrichtung, bei der das Fixierungselement und der Gummizug noch nicht befestigt sind,

Fig. 2 eine Seitenansicht der Vorrichtung mit befestigtem Fixierungselement und Gummizug,

Fig. 3 eine Seitenansicht der Schiene, und

Fig. 4 eine Ansicht einer weiteren Ausführungsform der Vorrichtung.

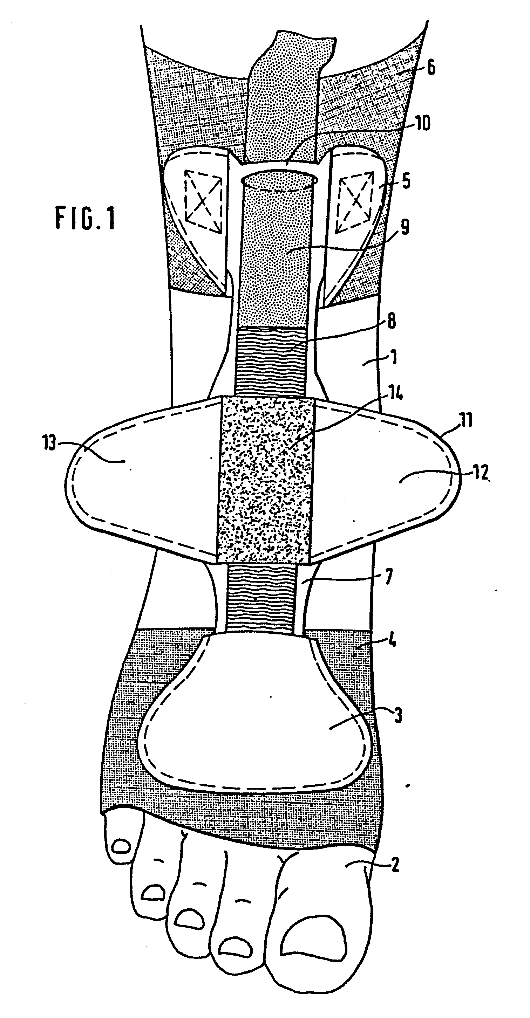

Fig. 1 zeigt eine über einen Fuß 2 angezogene Socke 1, die aufgrund ihrer Elastizität stramm am Fuß anliegt. Die Elastizität wird in bei Stützstrümpfen bekannter Weise durch einarbeiten von quer- und längsverlaufenden Gummifäden erreicht. Am vorderfußseitigen Ende ist die Socke 1 offen, um zu verhindern, daß einerseits die Zehen durch die fest anliegende Socke in ihrer Bewegungsfreiheit eingeengt werden, und daß andererseits ein frühzeitiger Verschleiß der Socke durch die Zehennägel erfolgt. Auf der fußrückenseitigen Oberfläche der Socke ist eine Tasche 3 aufgesteppt. In dem die Tasche 3 umgebenden Bereich 4 ist das Gewebe der Socke verstärkt ausgeführt, damit die Tasche 3 ausreichenden Halt findet. Eine zweite Tasche 5 ist an der schienbeinseitigen Oberfläche der Socke aufgesteppt, die in diesem Bereich 6 ebenfalls verstärkt ist. In die Taschen 3 und 5 greifen die Enden einer aus einem elastisch nachgiebigen Material (z. B. aus dem unter dem Handelsnamen "Ortholen" bekannten Kunststoff oder aus Stahl) angefertigten Schiene 7 ein. Dabei können die Enden der Schiene wie auch die Taschen so ausgebildet sein, daß die Schiene nach dem Aufsteppen der Taschen 3 und 5 fest mit dem Strumpf verbunden ist. Diese Möglichkeit ist in der Zeichnung dargestellt. Statt dessen kann die Form der Schienenenden und der Taschen aber auch so aufeinander abgestimmt werden, daß die Schiene in die Taschen einsteckbar ist. In der Praxis hat sich gezeigt, daß die Möglickeit des Einsteckens die Handhabung erleichtert, denn eine fest mit der Socke verbundene Schiene erschwert das Anziehen der Socke, während andererseits das Einstecken der Schiene in die elastische Socke nach dem Anziehen keine Schwierigkeiten bereitet. Im letzteren Fall ist es jedoch erforderlich, ein Herausrutschen der Schiene aus der Tasche 5 beim Gehen durch die Beuge- oder Streckbewegung des Unterschenkels zu verhindern. Hierzu kann eine nicht dargestellte Verschlußvorrichtung vorgesehen werden, die beispielsweise aus zwei die Schiene 7 im Bereich der Tasche 5 übergreifenden Bändern besteht, die als Klett- oder Druckverschluß ausgebildet sind.

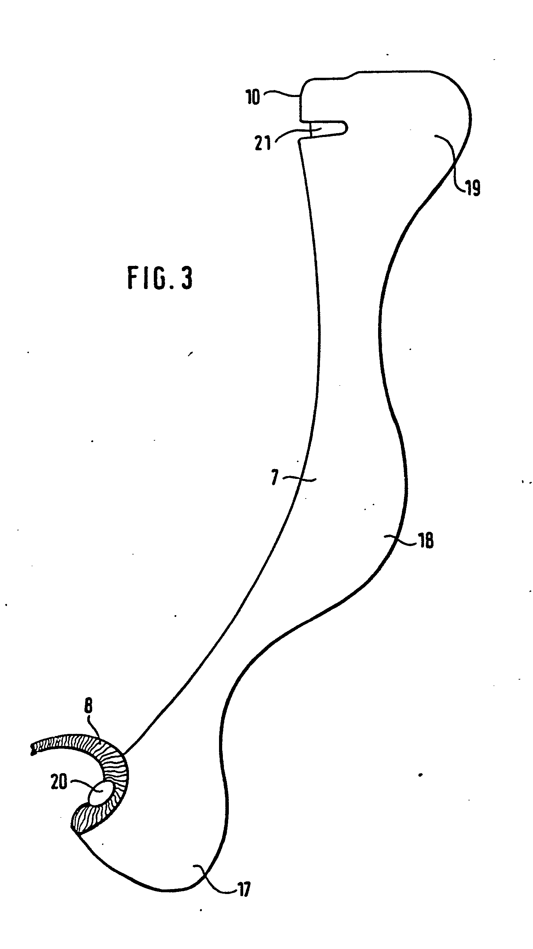

Am vorderfußseitigen Ende der Schiene 7 ist - wie aus Fig. 3 ersichtlich - ein Gummizug 8 befestigt, der durch ein Klettband 9 verlängert ist. Der Gummizug 8 und das Klettband 9 verlaufen zum schienbeinseitigen Ende der Schiene 7, wo ein Schlitz 21 eingearbeitet ist, durch den ein Steg 10 gebildet wird, so daß das Klettband durch den Schlitz 21 um den Steg 10 gefädelt und wieder in Richtung auf das Fußende zurückgeführt werden kann. Der Gummizug 8 bzw. das Klettband 9 bilden praktisch eine Sehne zwischen den Enden der Schiene 7.

Im mittleren Bereich zwischen den Enden der Schiene 7 ist der Gummiring 8 durch ein Fixierungselement 11 hindurchgeführt. Die Länge des Gummizuges ist dabei so bemessen, daß er noch durch das Fixierungselement 11 hindurchverläuft. Das Fixierungselement 11 ist beiderseits des Gummizuges 8 mit je einer seitlich abstehenden Lasche 12, 13 versehen, wobei auf der Oberseite des Fixierungselementes zwischen den Laschen 12 und 13 ein mittlerer Bereich 14 vorgesehen ist, der ausreichend flauschig ausgebildet ist, um das hinter dem Steg 10 zurückgeführte Klettband 9 daran befestigen zu können. Das Fixierungselement 11 ist auf dem Gummizug 8 zunächst frei verschieblich, jedoch sind die der Socke 1 zugekehrten Flächen der Laschen 12 und 13 mit Klettmaterial versehen, so daß damit die Laschen 12 und 13 an der Socke festgelegt werden können.

Fig. 1 zeigt den Zustand, bei dem die Socke über den Fuß gestreift worden ist, bei dem aber noch nicht der Vorderfuß in seine abgehobene Stellung gebracht worden ist.

Die Benutzung der erfindungsgemäßen Vorrichtung geht folgendermaßen vor sich. Nachdem die Socke 1 angezogen und gegebenenfalls die Schiene 7 in die Taschen 3 und 5 eingesteckt worden ist, wird das die Verlängerung des durch das Fixierungselement 11 hindurchgeführten Gummizuges bildenede Klettband 9 durch den Schlitz 21 (Fig. 3) hindurchgefädelt und um den Steg 10 bis zum Bereich 14 des Fixierungselementes 11 zurückgeführt, wobei der Gummizug 8 so gespannt wird, daß der Fuß in seine Normallage angehoben wird, bei der er praktisch einen rechten Winkel zum Unterschenkel bildet. Mit dieser Vorspannung wird das Klettband an den Bereich 14 des Fixierungselementes angedrückt und damit der Fuß in dieser Normallage festgelegt.

Die selbsthemmende Wirkung des Klettbandes im Bereich des Steges 10 der Schiene 7 sorgt dabei dafür, daß das Fixierungselement 11 der Spannung des Gummizuges 8 nicht nachgibt. Gegebenenfalls muß aber das Fixierungselement 11 beim Festlegen des Klettbandes 9 von Hand festgehalten werden.

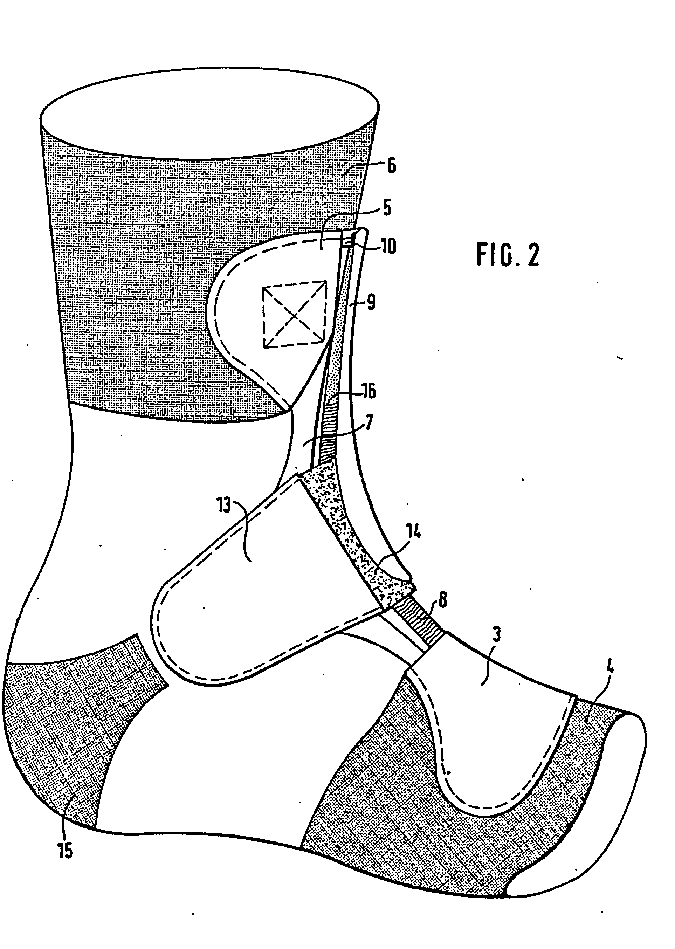

Anschließend daran werden die Laschen 12 und 13 des Fixierungselementes in Richtung auf den Fußrücken gezogen und dann gemäß Fig. 2 an die Socke 1 angedrückt und durch das Klettmaterial an dieser verhaftet. Hierdurch wird zweierlei erreicht, nämlich einerseits, daß der Gummizug praktisch der Form der Schiene 7 angepaßt wird und aus dem Weg kommt, und andererseits, daß der Gummizug zusätzlich gespannt wird, wodurch der Vorderfuß etwas über seine Normallänge hinaus angehoben wird.

Diese Anhebung ist nützlich, wenn anschließend ein Schuh angezogen wird, weil dann diese zusätzliche Anhebung des Vorderfußes durch das Gesicht des Schuhes wieder rückgängig gemacht wird.

In Fig. 2 ist zu erkennen, daß auch im Fersenbereich der Socke noch eine Verstärkung 15 angebracht ist, die dazu dient, die durch das Fixierungselement 11 ausgeübten Kräfte aufzunehmen.

Aus Fig. 2 ist ersichtlich, daß im Endzustand die erfindungsgemäße Vorrichtung kaum aufträgt, so daß normales Schuhwerk getragen werden kann, was für den Erkrankten sowohl in praktischer wie auch in kosmetischer Hinsicht ein außerordentlicher Vorteil ist.

Die Seitenansicht der Schiene 7 in Fig. 3 zeigt, daß die Schiene so ausgebildet ist, daß sie in den Bereichen 17, 18 und 19 mit verbreiterten Wölbungen versehen ist, die der anatomischen Form des Fußrückens angepaßt sind, so daß die Schiene damit eine stark stabilisierende Funktion ausüben kann, die auch bei Funktionsstörungen des Peronaeusnervs gelegentlich auftretenden seitlichen Auslenkungen des Fußes stabilisierend entgegenwirken. Die Bereiche 17 und 19 werden von den beiden auf der Socke befestigten Taschen 3 und 5 aufgenommen. Zur Befestigung des Gummizuges 8 dient ein Niet 20 oder dergleichen.

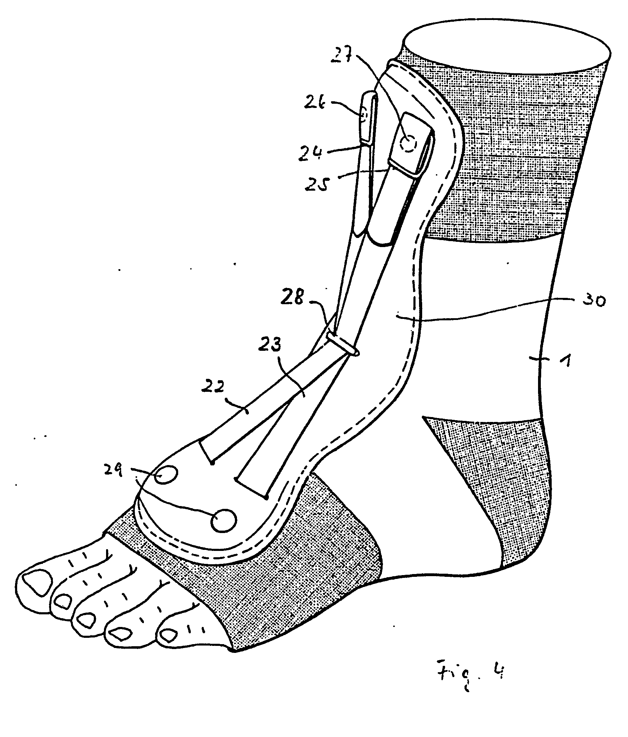

Bei dem in Fig. 4 dargestellten Ausführungsbeispiel ist die Schiene 7 auf ihrer gesamten Länge fest mit der Socke 1 verbunden. Zur Befestigung dient eine Lederlasche 30, die die Schiene voll überdeckt und auf die Socke aufgenäht ist, so daß die Schiene nicht sichtbar ist.

Der Gummizug besteht bei diesem Ausführungsbeispiel aus zwei Gummibändern 22 und 23, die mit seitlichem Abstand voneinander an dem vorderfußseitigen Ende der Schiene befestigt sind, z. B. durch Nieten 29. An den schienbeinseitigen Enden der Gummibänder 22 und 23 ist je eine Schnalle 24 bzw. 25 angebracht. Die Schnallen sind nach Art von Gürtelschnallen ausgebildet, so daß ihre Lage auf den Gummibändern veränderbar und anschließend durch Umfalten festlegbar ist. An den Schnallen 24 und 25 befindet sich der eine Teil eines Druckknopfes 26 bzw. 27, während die beiden anderen Teile der Druckknöpfe im Abstand voneinander an der Lederlasche befestigt sind.

An der Schiene ist in ihrem mittleren Bereich eine Öse 28 angebracht, durch die die beiden Gummibänder 22 und 23 hindurchgefädelt sind, so daß sie im Bereich der Öse in unmittelbarer Nähe der Schiene verlaufen.

Die Gummibänder 22 und 23 sind von dem einen Ende der Schiene zum anderen Ende über Kreuz geführt.

Die Verwendung von zwei Gummibändern hat den Vorteil, daß an beiden Enden der Schiene jeweils zwei im Abstand voneinander liegende Angriffspunkte für die Zugkraft vorhanden sind, so daß sich für den Fuß eine bessere seitlichen Stabilisierung ergibt.

**Patentansprüche**

1. Vorrichtung zur Anhebung des Vorderfußes bei einer Funktionsstörung des Peronaeusnervs unter Verwendung einer zwischen dem Vorderfuß und dem Unterschenkel wirkenden elastischen Verbindung (8, 22, 23) mit einstellbarer Vorspannung, dadurch gekennzeichnet, daß die Vorrichtung aus einer fest am Fuß anliegenden Socke (1) besteht, auf deren fußrückenseitiger Oberfläche eine Schiene (7) befestigt ist, welche aus einem elastisch nachgiebigen Material dem Fußrücken anatomisch angepaßt gefertigt ist und zwischen Vorderfuß und unterem Schienbeinbereich angeordnet ist, und daß die elastische Verbindung (8, 22, 23) an den Enden der Schiene (7) angreift.

2. Vorrichtung nach Anspruch 1, dadurch gekennzeichnet, daß die Schiene (7) mit ihren beiden Enden in zwei mit der Socke (1) fest vernähten Taschen (3, 5) sitzt.

3. Vorrichtung nach Anspruch 2, dadurch gekennzeichnet, daß die Schiene (7) in die Taschen (3, 5) einsteckbar ist.

4. Vorrichtung nach einem der Ansprüche 1 - 2, dadurch gekennzeichnet, daß die Socke (1) aus einem elastischen Gewebe besteht, das im Fersenbereich (15) und im Bereich der beiden Taschen (3, 5) verstärkt ist.

5. Vorrichtung nach einem der Ansprüche 1 - 4, dadurch gekennzeichnet, daß die in ansich bekannter Weise als Gummizug (8) ausgebildete elastische Verbindung in einem mittleren Bereich zwischen den Taschen (3, 5) mittels eines Fixierungselements (11) geführt ist, das beiderseits des Gummizuges (8) mit je einer seitlich abstehenden Lasche (12, 13) verbunden ist, daß der Gummizug (8) oberhalb des Fixierungselements (11) durch ein Klettband (9) verlängert ist, und daß das freie Ende des Klettbandes (9) um einen Steg (10) der in die Schiene (7) am schienbeiseitigen Ende eingearbeitet ist, zum Fixierungselement (11) zurückgeführt und daran befestigt ist.

6. Vorrichtung nach Anspruch 5, dadurch gekennzeichnet, daß das Fixierungselement (11) an den beiden der Socke (1) zugewandten Laschenoberflächen mit Klettmaterial versehen und dadurch auf der Socke (1) festlegbar ist.

7. Vorrichtung nach Anspruch 1, dadurch gekennzeichnet, daß die in ansich bekannter Weise als Gummizug ausgebildete elastische Verbindung aus zwei Gummibändern (22, 23) besteht, die mit ihrem einen Ende mit seitlichem Abstand voneinander an dem vorderfußseitigen Ende der Schiene (7) starr befestigt sind und an ihrem anderen Ende jeweils eine verstellbare Schnalle (24 bzw. 25) mit dem einen Teil eines Druckknopfes (26 bzw. 27) aufweisen, der auf die anderen Teile der Druckknöpfe, die an dem schienbeinseitigen Ende der Schiene in seitlichem Abstand voneinander angebracht sind, aufrastbar ist, und daß die beiden Gummibänder (22, 23) vom einen Ende der Schiene (7) zum anderen Ende über Kreuz geführt sind.

8. Vorrichtung nach Anspruch 7, dadurch gekennzeichnet, daß im mittleren Bereich der Schiene ein Öse (28) angebracht ist, durch die die Gummibänder (22, 23) in unmittelbarer Nähe an der Schiene hindurchgeführt sind.

9. Vorrichtung nach Anspruch 8, dadurch gekennzeichnet, daß die Schiene (7) auf ihrer ganzen Länge an der Socke (1) befestigt ist.

10. Vorrichtung nach Anspruch 9, dadurch gekennzeichnet, daß die vorderfußseitigen Enden der Gummibänder (22, 23) mit der Schiene (7) vernietet sind.

**Claims**

1. Device for raising the forefoot in the case of a functional defect of the peroneus nerve using an elastic connection (8, 22, 23) with adjustable pretensioning acting between the forefoot and the lower leg, characterised in that the device consists of a sock (1) which is held firmly against the foot and to the surface of which on the instep side of the foot is secured a splint (7) which is formed from an elastically pliable material which matches the instep anatomically and is arranged between the forefoot and the lower shin-bone area and in that the elastic connection (8, 22, 23) engages at the ends of the splint (7).

2. Device according to claim 1, characterised in that the splint (7) is seated at its two ends in two pockets (3, 5) which are sewn firmly to the sock (1).

3. Device according to claim 2, characterised in that the splint (7) can be inserted into the pockets (3, 5).

4. Device according to one of claims 1 to 2, characterised in that the sock (1) consists of an elastic fabric which is reinforced in the heel area (15) and in the area of the two pockets (3, 5).

5. Device according to one of claims 1 to 4, characterised in that the elastic connection formed as an elastic strip (8) in a manner known in itself is run in a central area between the pockets (3, 5) using an attachment part (11) which is connected on each side of the elastic strip (8) in each case by a flap (12, 13) protruding at the side, in that the elastic strip (8) is extended above the attachment part (11) by means of an adhesive strip (9) and in that the free end of the adhesive strip (9) is drawn back to the attachment part (11) and secured to it about a crosspiece (10) which is worked into the splint (7) at the shin-bone end.

6. Device according to claim 5, characterised in that the attachment part (11) is provided with adhesive material at the two flap surfaces turned towards the sock (1) and in this way can be fastened to the sock (1).

7. Device according to claim 1, characterised in that the elastic connection formed as an elastic strip in a manner known in itself consists of two elastic strips (22, 23), the ends of which on one side with a lateral clearance from each other are attached rigidly to the end of the splint (7) on the forefoot side and at their other ends in each case are provided with an adjustable buckle (24, 25) with one part of a press fastener (26, 27) which can be locked onto the other parts of the press fasteners which are fitted at the end of the splint on the shin-bone side with a lateral clearance and in that the two elastic strips (22, 23) are run in the form of a cross from one end of the splint (7) to the other.

8. Device according to claim 7 <u>characterised in that</u> an eyelet (28) is fitted in the central area of the splint through which the elastic strips (22, 23) are passed in the immediate vicinity of the splint.

9. Device according to claim 8, <u>characterised in that</u> the splint (7) is attached to the sock (1) over its entire length.

10. Device according to claim 9, <u>characterised in that</u> the ends of the elastic strips (22, 23) on the forefoot side are riveted to the splint (7).

**Revendications**

1. Appareil pour relever un pied ballant en cas de défaillance du système nerveux du péroné, utilisant une liaison élastique (8, 22, 23) agissant entre le pied et la jambe avec une tension initiale réglable, <u>caractérisé en ce que</u> l'appareil consiste en une chaussette (1) serrant fermement le pied, sur la partie de la surface de laquelle, qui recouvre le cou-de-pied est fixée une attelle (7) en matière souple et élastique, qui est adaptée à l'anatomie du cou-de-pied et placée entre le pied et la partie inférieure du tibia, et en ce que la liaison élastique (8, 22, 23) s'accroche aux extrémités de l'attelle (7).

2. Appareil suivant la revendication 1, <u>caractérisé en ce que</u> l'atelle (7) est tenue par ses deux extrémités dans deux poches (3, 5) piquées sur la chaussette (1).

3. Appareil suivant la revendication 2, <u>caractérisé en ce que</u> l'attelle (7) est enfichable dans les poches (3, 5).

4. Appareil suivant la revendication 1 ou 2, <u>caractérisé en ce que</u> la chaussette (1) est en tissu élastique qui est renforcé autour du talon (15) et des deux poches (3, 5).

5. Appareil suivant l'une des revendications 1 à 4, <u>caractérisé en ce que</u> la liaison élastique, formée d'une manière connue en soi d'une bande élastique (8), est guidée, dans la partie médiane entre les poches (3, 5) au moyen d'un élément de fixation (11), qui est respectivement, de part et d'autre de la bande élastique (8), reliée à des languettes latérales (12, 13) et en ce que la bande élastique (8) est prolongée, au-dessus de l'élément de fixation (11), par une bande auto-agrippante (9) et en ce que l'extrémité libre de la bande auto-agrippante (9) passe autour d'une barrette (10) incorporée à l'extrémité de l'attelle (7) qui se trouve du côté du tibia, et revient vers l'élément de fixation (11) auquel elle est fixée.

6. Appareil suivant la revendication 5, <u>caractérisé en ce</u> les deux surfaces, faisant face à la chaussette, des languettes de l'élément de fixation (11) sont recouvertes d'une matière auto-agrippante et que de ce fait, ce dernier peut être fixé sur la chaussette (1).

7. Appareil suivant la revendication 1, <u>caractérisé en ce que</u> la liaison élastique, formée d'une manière connue en soi d'une bande élastique, comprend deux bandes élastiques (22, 23) qui sont fixées par leurs premières extrémités, avec un écart latéral entre elles, à l'extrémité de l'attelle (7) qui se trouve du côté du cou-de-pied, et qui comportent respectivement, à leurs autres extrémités, des boucles (24 ou 25) avec les premières parties de boutons-pression (26 ou 27) qui se fixent sur les autres parties des boutons-pression, lesquelles sont montées avec un écart latéral entre elles, sur l'extrémité de l'attelle (7) qui se trouve du côté du tibia, et en ce que les deux bandes élastiques (22, 23) passent d'une extrémité de l'attelle (7) à l'autre en se croisant.

8. Appareil suivant la revendication 7, <u>caractérisé en ce que</u>, dans la partie médiane de l'attelle (7), est fixé un anneau (28) dans lequel passent les bandes élastiques (22, 23) à proximité immédiate de l'attelle.

9. Appareil suivant la revendication 8, <u>caractérisé en ce que</u> l'attelle (7) est fixée sur toute sa longueur sur la chaussette (1).

10. Appareil suivant la revendication 9, <u>caractérisé en ce que</u> les extrémités des bandes élastiques (22, 23), qui se trouvent du côté du cou-de-pied, sont rivetées sur l'attelle (7).

FIG.1

FIG. 2

FIG. 3

Fig. 4